# EUROPEAN PATENT SPECIFICATION

(11) **EP 1 164 953 B1**
(45) Date of publication and mention of the grant of the patent: **24.10.2007**
(21) Application number: 00908890.7
(22) Date of filing: 17.03.2000
(51) Int. Cl.: A61B 17/70

(54) **TRANSCONNECTOR FOR COUPLING SPINAL RODS**
QUERVERBINDUNG ZUM VERBINDEN VON WIRBELSÄULENSTÄBEN
TRANSCONNECTEUR POUR LE COUPLAGE DE BROCHES SPINALES

(30) Priority: 06.04.1999 US 286669; 17.12.1999 US 466725
(43) Date of publication of application: 02.01.2002
(73) Proprietor: Synthes GmbH, 4436 Oberdorf (CH)
(72) Inventor: TROXELL, Thomas, N., Pottstown, PA 19465 (US); KUMAR, G., Kris, West Chester, PA 19382 (US); RUNCO, Thomas, J., Devon, PA 19333 (US); DAVIS, Barclay, R., Downingtown, PA 19335 (US)
(74) Representative: Lusuardi, Werther
(86) International application number: PCT/CH2000/000153
(87) International publication number: WO 2000/059387

(56) References cited:
- EP-A- 0 514 303
- WO-A-97/38640
- US-A- 5 275 600
- US-A- 5 624 442

## Description

The present invention relates to a transconnector for coupling first and second elongate spinal fixation elements according to the definition of claim 1.

It is often necessary to surgically treat spinal disorders such as scoliosis. Numerous systems for use in spinal correction and fixation have been disclosed. These systems usually include a pair of elongate members, typically either rods or plates, placed along the vertebral column. For the sake of simplicity, the term "rod" is used throughout to refer to any elongate member. Each rod is attached to the spine with various attachment devices. These attachment devices may include, but are not limited to, pedicle screws, plates, transverse process hooks, sublaminar hooks, pedicle hooks, and other similar devices.

It is also well known that the strength and stability of the dual rod assembly can be increased by coupling the two rods with a cross-brace or transconnector which extends substantially horizontal to the longitudinal axes of the rods across the spine. The simplest situation in which a transconnector could be used occurs when the two rods are geometrically aligned. Specifically, the two rods are parallel to each other, i.e. there is no rod convergence or divergence in the medial-lateral direction; the two rods have the same orientation with respect to the coronal plane (viewed in the anterior-posterior direction), i.e the rods are coplanar from a lateral view; and the two rods are located a uniform distance from each other.

Due to a wide variety of factors, the two rods are rarely three dimensionally geometrically aligned in clinical situations. There are several ways to address the variations of geometrical alignment. First, one or both of the rods can be bent to accommodate the transconnector. However, any bending in either of the rods can adversely affect the fixation to the spine and comprise clinical outcome. Furthermore, the bending can also adversely affect the mechanical properties of the rods. The transconnector can also be bent so that the disturbance to the rod positioning is minimized. As is the case with bending of the rods, the mechanical properties of the transconnector could be compromised.

Transconnectors with some adjustability have been designed to adapt for variations from geometrical alignment. However, most are multi-piece systems that can be difficult to assemble and use in the surgical environment. U.S. Patent No. 5,980,523 discloses a muti-piece transverse connector for spinal rods that can accommodate converging or diverging rods. However, accidental disassembly of the connector by the surgeon is possible. Even those that are one-piece designs do not allow for adjustments to compensate for all three modes in which there may be variation from geometrical alignment: convergence or divergence, non-coplanar rods, and variability in rod separation distances. For example, U.S. Patent No. 5,947,966 discloses a device for linking adjacent spinal rods. In one embodiment, the device includes two members that are movable with respect to one another to accommodate different rod separation distances. A pin on one member engages a groove on the other member to provisionally couple the two members, thereby preventing a surgeon from separating the two members. Because the pin is sized to exactly fit the groove, no movement of the pin transverse to the longitudinal axis of the groove is possible. As a result, the device disclosed in the '966 patent cannot accommodate non-coplanar rods or adjust for rod convergence or divergence.

Similarly, U.S. Patent No. 5,275,600 also discloses a device for linking adjacent spinal rods. This device includes two members that are moveable with respect to each other to accommodate different rod separation distances and rods that are non-coplanar. A projection on one member is inserted into a cavity in the second member and a screw operatively engaged with the second member abuts the projection to prevent uncoupling. Although this device can accommodate different rod separations and rods that are non-coplanar, this device cannot accommodate rods that converge or diverge since the projection in the cavity of the second member can only rotate axially and move along a single axis.

Thus, there exists a need for an improved transconnector for coupling spinal rods.

The present invention relates to a transconnector for coupling first and second elongate spinal fixation elements that have different three dimensional orientations. The transconnector includes a male member, a female member and a locking member and can be made of any suitable material such as titanium, a titanium alloy, or stainless steel. The male member comprises a body with lateral and medial ends, a linking element associated with the lateral end and being configured and dimensioned to receive one of the fixation elements, and a projection on the medial end. The projection includes a body with a recess defined by lateral and medial walls. The female member comprises a body with lateral and medial ends, a linking element associated with the lateral end and being configured and dimensioned to receive one of the fixation elements, and a cavity with an opening on the medial end which is configured and dimensioned to receive a portion of the male member projection. The locking member secures the position and orientation of the male member projection portion in the cavity in order to accommodate different separation distances and orientations between the first and second fixation elements. The locking member interacts with the medial wall of the recess to limit travel of the male member projection portion in the cavity, thereby preventing uncoupling of the male and female members.

Preferably, the locking member comprises a threaded hole in the body of the female member and a set screw threadably received in the threaded hole. The set screw has a first end for receiving a tool to turn the set screw and a second end contactable with the projection for pressing the projection against the cavity. The recess may have a width wider than a width of the second end of the set screw for the rotation of the projection in the cavity. In an exemplary embodiment, the projection has a substantially cylindrical shape with a first radius and the recess has a curved upper surface with a second radius. The first radius is larger than the second radius and the second end of the set screw extends into the recess to limit the rotation of the projection in the cavity. The set screw can be a threaded cylindrical body transitioning to a tip at the second end of the set screw.

The male member body may comprise a link terminal having a lateral end with the male member linking element, an intermediate link having a medial end with the projection of the male member and a lateral end engaging the medial end of the link terminal, and a locking element for securing the link terminal to the intermediate link. Preferably, the medial end of the link terminal includes a first textured surface and the lateral end of the intermediate link includes a second textured surface mating with the first textured surface. The first textured surface is rotatable with respect to the second textured surface for accommodating convergence or divergence between the first and second rods. An example of suitable first and second textured surfaces includes a radial or star-grind pattern.

The locking element preferably comprises a first hole through the medial end of the link terminal, a second hole through the lateral end of the intermediate link aligned with the first hole, and a cap screw insertable in the first and second holes. The cap screw may have a second end with a retaining ring for preventing removal of the cap screw from the second hole. The retaining ring may include a resilient member which flexes inward upon insertion of the cap screw through the second hole and flexes outward once the resilient member is past a collar in the second hole. Preferably, the resilient member includes an end of the cap screw with a lip and a plurality of slits.

If rods are used for the elongate fixation elements, then the male member linking element preferably comprises a hook and the female member linking element preferably comprises a hook. The lateral ends of the male and female members each may include a threaded hole and a clamping screw threadably received in the respective threaded hole for securing the fixation elements to the respective hook. The fixation elements are preferably clamped between a conical second body portion of the respective clamping screw and a region near the tip portion of the respective hook when the transconnector is secured to the fixation elements.

Preferred features of the present invention are disclosed in the accompanying drawings, wherein similar reference characters denote similar elements throughout the several views, and wherein:
FIG. 1 shows a top perspective view of a transconnector according to the present invention with an elongate fixation element attached at each end;
FIG. 2 shows a bottom perspective view of the transconnector without the fixation elements;
FIG. 3 shows a cross-sectional view of the transconnector with one fixation element attached;
FIG. 4 shows a perspective view of a portion of one embodiment of the male member;
FIG. 5 shows a cross-section of the male member taken through line 5-5 of FIG. 4;
FIG. 6 shows a perspective view of the female member of the transconnector;
FIG. 7 shows a side view of the female member;
FIG. 8 shows a side view of a link terminal of the male member with a partial cross section;
FIG. 9 shows a cross-sectional view of an intermediate link of the male member; and
FIG. 10 shows a perspective view of a cap screw used to join the link terminal and intermediate link.

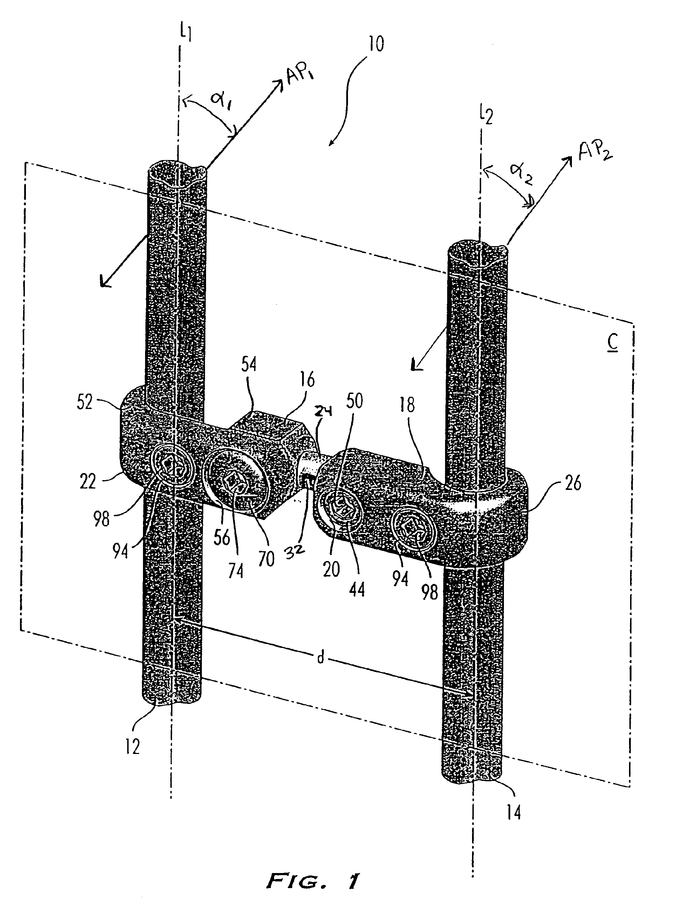

FIG. 1 shows a transconnector 10 according to the present invention for coupling a first elongate spinal fixation element 12 to a second elongate spinal fixation element 14. Transconnector 10 can be made of any suitable material typically used in orthopaedic applications such as titanium, titanium alloy, or stainless steel. If transconnector 10 is made of a metallic material, preferably it is the same metallic material used for fixation elements 12, 14 to avoid galvanic (mixed-metal) corrosion. First and second fixation elements 12, 14 can be cylindrical rods, rectangular bars, plates, or any other device suitable for spinal fusion. In use, first fixation element 12 extends along one side of the vertebral column and second fixation element 14 extends along the other side of the vertebral column. A wide variety of attachment devices such as hooks, screws, and clamps, can be used to attach first and second fixation elements 12, 14 to the spine.

Transconnector 10 includes a male member 16, a female member 18, and a locking member 20. Male member 16 has a body with a linking element 22 on the lateral end for receiving first fixation element 12 and a projection 24 extending from the medial end of the body. Female member 18 has a body with a linking element 26 on the lateral end for receiving second fixation element 14 and a cavity 28 with an opening 30 (FIGS. 6 and 7) on the medial end of the body for receiving a portion of projection 24. Locking member 20 secures the portion of projection 24 in cavity 28. The portion of projection 24 received in cavity 28 is adjustable for accommodating different separation distances d between first and second fixation elements 12, 14. This feature allows transconnector 10 to be readily adjusted for different patient anatomies and used in different regions of the spine. For example, the lumbar vertebrae are typically larger than the thoracic vertebrae. As a result, the distance between fixation elements in the lumbar region would be greater than fixation elements in the thoracic region. Because the length of projection that slides into cavity 28 can be varied, transconnector can be adjusted for use in different spinal regions without the need to bend either fixation rods or transconnector 10. In order to further increase the adjustability of transconnector 10, female member 18, cavity 28, and projection 24 can be manufactured in different sizes.

Projection 24 is rotatable in cavity 28 for accommodating differences between the angular orientation of first fixation element 12 with respect to the coronal plane and the angular orientation of second fixation element 14 with respect to the coronal plane. Specifically, first fixation element 12 has a longitudinal axis L₁ which runs at an angle α₁ with respect to Line AP₁, which is perpendicular to plane C, and second fixation element 14 has a longitudinal axis L₂ which runs at an angle α₂ with respect to Line AP₂, which is also perpendicular to plane C, a plane which divides the body in half from front to back. Because projection 24 can rotate in cavity 28, transconnector can be used in situations in which α₁ differs from α₂ without the need to bend either fixation element or transconnector 10.

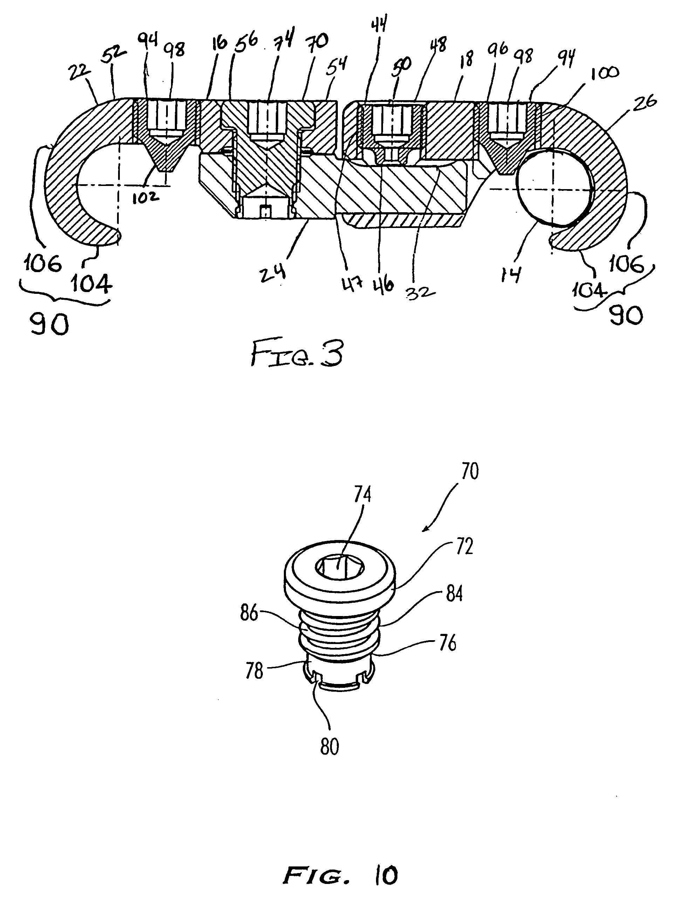

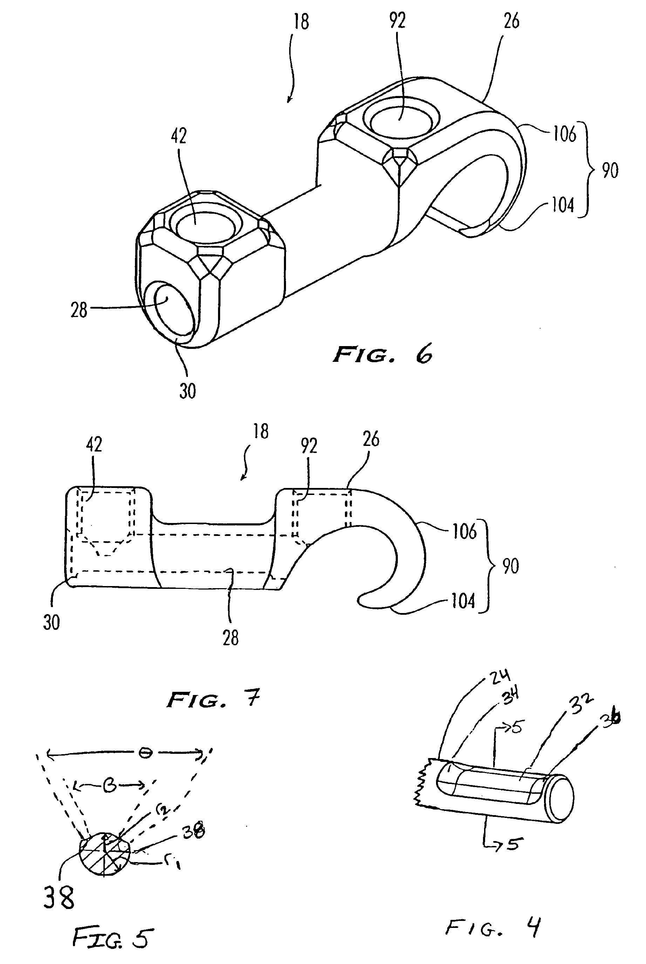

As seen best in FIGS. 3, 4, and 5, projection 24 has a substantially cylindrical-like shape and includes a recess 32 defined by lateral and medial walls 34, 36. As will be described in more detail below, medial wall 36 of recess 32 forms a stop against locking member 20 to limit travel of projection 24 in cavity 28, thereby preventing uncoupling of male member 16 and female member 18. Recess 32 can be formed having a number of different configurations (e.g. a flat planar surface) so long as medial wall 36 contacts locking member 20 before projection 24 is completely removed from cavity 28 and projection 24 can rotate within cavity 28. FIGS. 4, 5, and 9 show an exemplary embodiment of recess 32. A portion of an upper surface of recess 32, defined by angle β, has a reduced radius r₂ compared to the radius r₁ outside of the area defined by angle β. Arcuate surfaces 38 serve as a transition region from reduced radius r₂ to radius r₁. Radius r₂ and arcuate surfaces 38 can be manufactured using a number of different techniques. For example, they can be machined out by cutting, ball milling, or other milling processes.

As previously noted, locking member 20 secures the portion of projection 24 in cavity 28. FIGS. 1, 3, 6, and 7 show that locking member 20 includes a threaded hole 42 in female member 18 and a set screw 44. Set screw 44 threads into threaded hole 42 and a second end 46 presses projection 24 to clamp it against the walls of cavity 28. In an exemplary embodiment, set screw 44 comprises a threaded cylindrical body 47 that transitions to a tip at second end 46. A first end 48 of set screw 44 has an appropriately shaped and sized hole 50 (or a slot) for receiving a surgical instrument like a screwdriver to turn set screw 44.

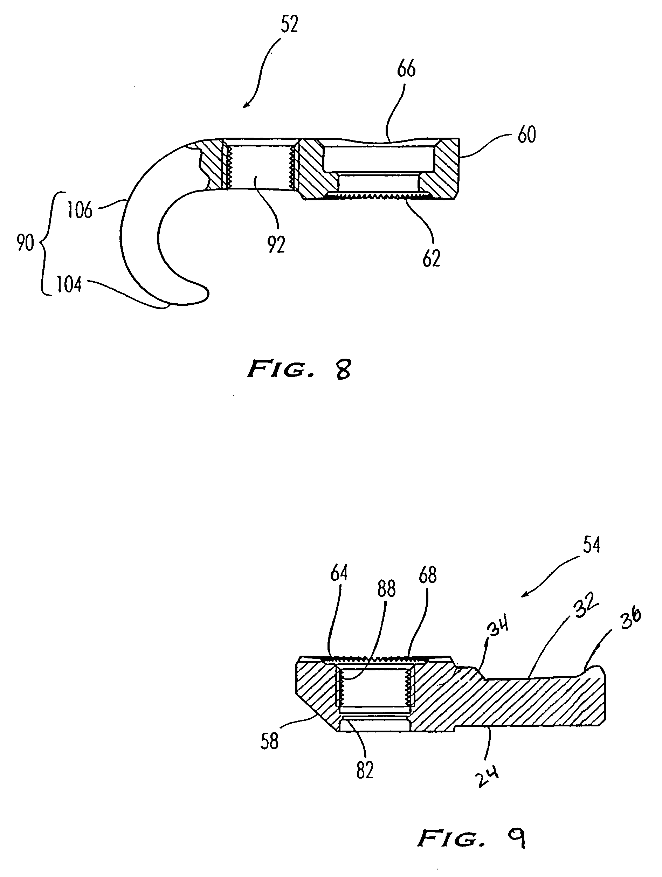

As also previously noted, projection 24 can rotate in cavity 28 to accommodate different orientations of first and second fixations elements 12, 14. The degree of possible rotation is determined by the geometries of recess 32 and set screw 44. Specifically, projection 24 can rotate within recess 32 until set screw 44 engages the edges of recess 32. With respect to the embodiment shown in FIGS. 4 and 5, angle 0, which includes the area of angle β and arcuate surfaces 38, defines the amount of rotation of projection 24 that is possible until second end 46 of set screw 44 engages the edges of recess 32 (that has the larger radius r₁).

In summary, recess 32 and locking member 20 interact to permit accommodation of non-coplanar rods and variability in rod separation distances. When locking member 20 includes set screw 44, set screw 44 can be in two basic positions. In the clamped position, set screw 44 is threaded into threaded hole 42 so that second end 46 presses against recess 32 to clamp projection 24 against the walls of cavity 28. In the unclamped position, movement of projection 24 within cavity 28 is permitted. The range of movement is limited by contact between set screw 44 and the boundaries of recess 32. In one embodiment, set screw 44 can be completely removed from threaded hole 42, thereby allowing projection 24 to completely leave cavity 28 and complete uncoupling of male and female members 16, 18. In another embodiment, set screw 44 is "staked" so that set screw 44 can not come out of threaded hole 42 without an increase in torque. One way to achieve this is by mechanically deforming the first threads of threaded hole 42 so that set screw 44 cannot be unscrewed past the damaged threads. It should also be noted that depending on the relative geometries of the set screw 44 and medial wall 36, uncoupling of male and female members 16, 18 may still be possible even without complete removal of set screw 44 from threaded hole 42 regardless of whether set screw 44 is staked.

FIGS. 1, 8, and 9 show an exemplary embodiment of the body of male member 16 as a two piece assembly which includes a link terminal 52, an intermediate link 54, and a locking element 56 to secure the two together. A lateral end of link terminal 52 has male member linking element 22 and intermediate link 54 has a medial end with projection 24 and a lateral end 58 which engages a medial end 60 of link terminal 52. Medial end 60 of link terminal 52 includes a first textured surface 62 which mates with a second textured surface 64 of lateral end 58 of intermediate link 54 in such a fashion that first textured surface 62 is rotatable with respect to second textured surface 64 to accommodate for any convergence or divergence between first and second fixation elements 12, 14. First and second textured surfaces 62, 64 are provided with a plurality of teeth, such as a radial or star-grind pattern, in order to help maintain link terminal 52 at the desired angular orientation. Locking element 56 includes a first hole 66 through medial end 60 of link terminal 52 and a second hole 68 through lateral end 58. First and second holes 66, 68 align so that a cap screw 70 can be inserted therethrough.

Referring to FIGS. 8-10, cap screw 70 has a first end 72 with a slot 74 for receiving a tool to turn cap screw 70 and a second end 76 with a retaining ring 78 for preventing removal of cap screw 70 from first and second holes 66, 68. Retaining ring 78 has slits 80 which allow retaining ring to flex inward to be inserted through a collar 82 in second hole 68. Once retaining ring 78 is inserted past collar 82, retaining ring 78 flexes back outward so that cap screw 70 can not be completely screwed out of first and second holes 66, 68. A body 84 of cap screw 70 is provided with threads 86 which engage threads 88 on the walls of second hole 68.

The structure of linking elements 22, 26 will depend on the structure of fixation elements 12, 14. For example, if fixation rods 12, 14 are elongate plates, then linking elements 22, 26 are configured and dimensioned to receive elongate plates. Such configurations and configurations for other types of fixation elements are well known in the art. If fixation elements 12, 14 are cylindrical rods as shown in the drawings, then linking elements 22, 26 each comprises a hook 90. The lateral ends of male and female members 16, 18 each includes a threaded hole 92 and a clamping screw 94 threadably received in threaded hole 92 for securing first and second fixation elements 12, 14 to hook 90.

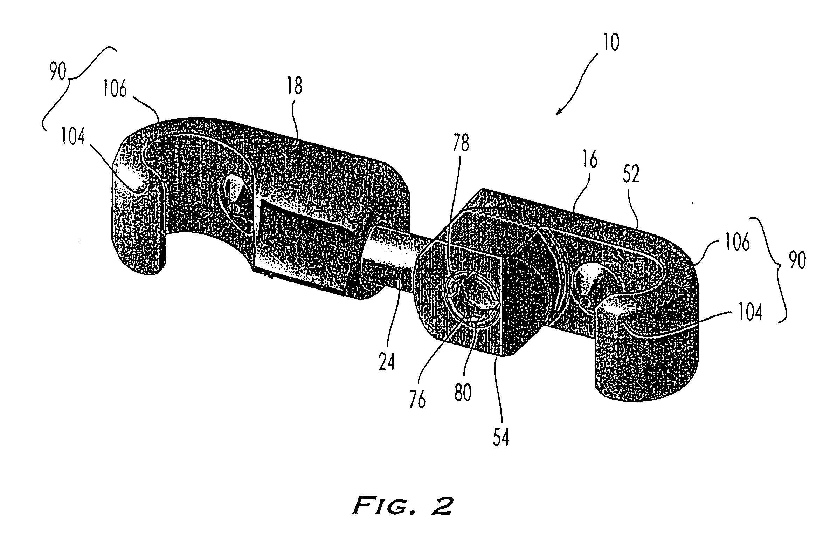

As seen best in FIGS. 2 and 3, each clamping screw 94 has a first end 96 with a slot 98 for receiving a tool to turn clamping screw 94, a threaded cylindrical first body portion 100, and a conical second body portion 102. Each hook 90 comprises a tip portion 104 and a curved portion 106. Curved portion 106 has a radius of curvature larger than the radius r of fixation elements 12, 14. As a result, the only contact between hooks 90 and fixation elements 12, 14 is at a region near tip portion 104. Furthermore, the only contact between clamping screws 94 and fixation elements 12, 14 is on conical second body portion 102. Thus, fixation elements 12, 14 is clamped between conical second body portion 102 and the region near tip portion 104.

While various descriptions of the present invention are described above, it should be understood that the various features can be used singly or in any combination thereof.

Therefore, this invention is not to be limited to only the specifically preferred embodiments depicted herein.

Further, it should be understood that variations and modifications within the scope of the invention may occur to those skilled in the art to which the invention pertains. Accordingly, all expedient modifications readily attainable by one versed in the art from the disclosure set forth herein that are within the scope of the present invention are to be included as further embodiments of the present invention. The scope of the present invention is accordingly defined as set forth in the appended claims.

## Claims

1. A transconnector (10) for coupling first and second elongate spinal fixation elements (12;14) that have different orientations, the transconnector (10)comprising:
A) a male member (16) comprising a body with lateral and medial ends, a linking element (22) associated with the lateral end and being configured and dimensioned to receive one of the fixation elements (12;14), and a projection (24) at the medial end, the projection (24) including a body with a recess (32) defined by lateral and medial walls (34;36);
B) a female member (18) comprising a body with lateral and medial ends and having a threaded hole (42), a linking element (26) associated with the lateral end and being configured and dimensioned to receive one of the fixation elements (12;14), and a cavity (28) with an opening (30) on the medial end which is configured and dimensioned to receive a portion of the male member projection (24);
**characterized in that** the transconnector further comprises
C) a set screw (44) for securing position and orientation of the male member projection portion in the cavity (28) in order to accommodate different separation distances and orientations between the first and second fixation elements (12;14), the set screw (44) threadably received in the threaded hole (42) and having a first end (48) for receiving a tool to turn the set screw (44) and a second end (46) contactable with the projection (24) for pressing the projection (24) against the cavity (28),
wherein interaction of the set screw (44) with the medial wall (36) of the recess (32) limits travel of the male member projection portion in the cavity (28) to thereby prevent uncoupling of the male and female members (16;18).

2. Transconnector of claim 1 wherein the recess (32) has a width wider than a width of the second end (46) of the set screw (44) to permit the rotation of the projection (24) in the cavity (28).

3. Transconnector of claim 2 wherein the projection (24) has a substantially cylindrical shape with a first radius and the recess (32) has a curved upper surface with a smaller second radius, wherein the second end (46) of the set screw (44) extends into the recess (32) to limit the rotation of the projection (24) in the cavity (28).

4. Transconnector of claim 1 wherein the set screw (44) comprises a threaded cylindrical body (47) transitioning to a tip at the second end (46) of the set screw (44).

5. Transconnector of claim 1 wherein the male member body comprises:
A) a link terminal (52) having a lateral end with the male member linking element (22) and a medial end;
B) an intermediate link (54) having a medial end with the projection (24) of the male member (16) and a lateral end engaging the medial end of the link terminal (52); and
C) a locking element (56) for securing the link terminal (52) to the intermediate link (54).

6. Transconnector of claim 5 wherein
A) the medial end (60) of the link terminal (52) includes a first textured surface (62);
B) the lateral end (58) of the intermediate link (54) includes a second textured surface (64) mating with the first textured surface (62); and
C) the first textured surface (62) is rotatable with respect to the second textured surface (64) for accommodating convergence or divergence between the first and second fixation elements (12;14).

7. Transconnector of claim 6 wherein the first and second textured surfaces (62;64) include a star-grind pattern.

8. Transconnector of claim 6 wherein the locking element (56) comprises:
A) a first hole (66) through the medial end (60) of the link terminal (52);
B) a second hole (68) through the lateral end (58) of the intermediate link (54) aligned with the first hole (66); and
C) a cap screw (70) insertable in the first and second holes (66;68).

9. Transconnector of claim 8 wherein the cap screw (70) has a first end (72) for receiving a tool to turn the cap screw (70) and a second end (76) with a retaining ring (78) for preventing removal of the cap screw (70) from the second hole (68).

10. Transconnector of claim 9 wherein the cap screw (70) has a body (84) with threads (86) and the second hole (66) is threaded for threadably receiving the cap screw (70).

11. Transconnector of claim 9 wherein the second hole (66) includes a collar (82) and the retaining ring (78) includes a resilient member, the resilient member flexing inward upon insertion of the cap screw (70) through the second hole (66) and flexing outward once the resilient member is past the collar (82) for preventing removal of the cap screw (70).

12. Transconnector of claim 11 wherein the resilient member includes an end of the cap screw (70) with a lip and a plurality of slits.

13. Transconnector of claim 1 wherein the male member linking element (22) comprises a hook (90) and the female member linking element (26) comprises a hook (90).

14. Transconnector of claim 13 wherein the lateral end of the male member (16) and the lateral end of the female member (18) each includes a threaded hole (92) and a clamping screw (94) threadably received in the respective threaded hole (92) for securing the fixation elements (12;14) to the respective hooks (90) of the male and female members (16;18).

15. Transconnector of claim 14 wherein each clamping screw (94) has a first end (96) for receiving a tool to turn the clamping screw (94), a threaded cylindrical first body portion (100), and a conical second body portion (102).

16. Transconnector of claim 15 wherein the hook (90) of the male member (16) and the hook (90) of the female member (18) each comprises a tip portion (104) and a curved portion (106) having a radius of curvature larger than that of fixation elements (12;14).

17. Transconnector of claim 16 wherein the fixation elements (12;14) are clamped between conical second body portion (102) of the respective clamping screw (94) and a region near the tip portion (104) of the respective hook (90) when the transconnector (10) is secured to the fixation elements (12;14).

18. Transconnector of claim 1 wherein the transconnector (10) is made of titanium, a titanium alloy, or stainless steel.

## Patentansprüche

1. Querverbinder (10) zur Verbindung von ersten und zweiten longitudinalen Fixationselementen (12;14) für die Wirbelsäule, welche unterschiedliche Orientierungen aufweisen, wobei der Querverbinder (10) umfasst
A) ein Steckelement (16), welches einen Körper mit lateralen und medialen Enden, ein mit dem lateralen Ende verbundenes, zur Aufnahme eines Fixationselementes (12;14) strukturiertes und dimensioniertes Verbindungselement (22) und einen Fortsatz (24) am medialen Ende umfasst, wobei der Fortsatz (24) einen Körper mit einer durch laterale und mediale Wände (34;36) definierten Vertiefung (32) beinhaltet;
B) ein aufnehmendes Element (18), welches einen Körper mit lateralen und medialen Enden umfasst und ein mit einem Gewinde versehenes Loch (42), ein mit dem lateralen Ende verbundenes, zur Aufnahme eines Fixationselementes (12;14) strukturiertes und dimensioniertes Verbindungselement (26) und einen Hohlraum (28) mit einer Öffnung (30) am medialen Ende hat, welcher zur Aufnahme eines Teils des Fortsatzes (24) am Steckelement strukturiert und dimensioniert ist;
**dadurch gekennzeichnet, dass** der Querverbinder ferner umfasst
C) eine Feststellschraube (44) zur Fixierung der Position und Orientierung des Teils des Fortsatzes am Steckelement im Hohlraum (28), um unterschiedliche Abstände und Orientierungen zwischen den ersten und zweiten Fixationselementen (12;14) anpassen zu können, wobei die Feststellschraube (44) mittels einer Gewindeverbindung in dem mit einem Gewinde versehenen Loch (42) aufgenommen wird, ein erstes Ende (48) zur Aufnahme eines Werkzeuges zum Drehen der Feststellschraube (44) und ein zweites, mit dem Fortsatz (24) in Kontakt bringbares Ende (46) zum Pressen des Fortsatzes (24) gegen den Hohlraum (28) aufweist,
wobei eine Wechselwirkung der Feststellschraube (44) mit der medialen Wand (36) der Vertiefung (32) die Bewegung des Teils des Fortsatzes (24) am Steckelement im Hohlraum (28) begrenzt, um so ein Entkuppeln des Steckelementes und des aufnehmenden Elementes (16;18) zu verhindern.

2. Querverbinder nach Anspruch 1, **dadurch gekennzeichnet, dass** die Vertiefung (32) eine grössere Breite aufweist als die Breite des zweiten Endes (46) der Feststellschraube (44), um so eine Rotation des Fortsatzes (24) im Hohlraum (28) zuzulassen.

3. Querverbinder nach Anspruch 2, **dadurch gekennzeichnet, dass** der Fortsatz (24) eine im wesentlichen zylindrische Form mit einem ersten Radius hat und die Vertiefung (32) eine gekrümmte obere Oberfläche mit einem kleineren zweiten Radius, wobei sich das zweite Ende (46) der Feststellschraube (44) in die Vertiefung (32) erstreckt, um die Rotation des Fortsatzes (24) im Hohlraum (28) zu begrenzen.

4. Querverbinder nach Anspruch 1, **dadurch gekennzeichnet, dass** die Feststellschraube (44) einen mit einem Gewinde versehenen zylindrischen Körper (47) umfasst, welcher am zweiten Ende (46) der Feststellschraube (44) in eine Spitze übergeht.

5. Querverbinder nach Anspruch 1, **dadurch gekennzeichnet, dass** der Körper des Steckelementes umfasst:
A) ein äusseres Verbindungsteil (52), welches ein laterales Ende mit dem Verbindungselement (22) des Steckelementes und ein mediales Ende hat;
B) ein dazwischenliegendes Verbindungsteil (54), welches ein mediales Ende mit dem Fortsatz (24) des Steckelementes (16) und ein laterales, in das mediale Ende des äusseren Verbindungsteils (52) eingreifendes Ende hat; und
C) ein Blockierelement (56) zur Fixierung des äusseren Verbindungsteils (52) am dazwischenliegenden Verbindungsteil (54).

6. Querverbinder nach Anspruch 5, **dadurch gekennzeichnet, dass**
A) das mediale Ende (60) des äusseren Verbindungsteils (52) eine erste, eine Struktur aufweisende Oberfläche (62) umfasst;
B) das laterale Ende (58) des dazwischenliegenden Verbindungsteils (54) eine zweite, eine Struktur aufweisende Oberfläche (64), welche in die erste, eine Struktur aufweisende Oberfläche (62) eingreift; und
C) die erste, eine Struktur aufweisende Oberfläche (62) relativ zur zweiten, eine Struktur aufweisenden Oberfläche (64) zur Einstellung der Konvergenz oder Divergenz zwischen den ersten und zweiten Fixationselementen (12;14) rotierbar ist.

7. Querverbinder nach Anspruch 6, **dadurch gekennzeichnet, dass** die erste und zweite, eine Struktur aufweisende Oberfläche (62;64) ein Sternschliff-Muster umfassen.

8. Querverbinder nach Anspruch 6, **dadurch gekennzeichnet, dass** das Blockierelement (56) umfasst:
A) ein erstes Loch (66) durch das mediale Ende (60) des äusseren Verbindungsteils (52);
B) ein zweites Loch (68) durch das laterale Ende (58) des dazwischenliegenden Verbindungsteils (54), welche mit dem ersten Loch (66) fluchtet; und
C) eine in das erste und zweite Loch (66;68) einführbare Kopfschraube (70).

9. Querverbinder nach Anspruch 8, **dadurch gekennzeichnet, dass** die Kopfschraube (70) ein erstes Ende (72) zur Aufnahme eines Werkzeuges zum Drehen der Kopfschraube (70) und ein zweites Ende (76) mit einem Haltering (78) zum Verhindern eines Entfernens der Kopfschraube (70) aus dem zweiten Loch (68) umfasst.

10. Querverbinder nach Anspruch 9, **dadurch gekennzeichnet, dass** die Kopfschraube (70) einen Körper (84) mit einem Gewinde (86) hat, und dass das zweite Loch (68) mit einem Gewinde versehen ist zur schraubbaren Aufnahme der Kopfschraube (70).

11. Querverbinder nach Anspruch 9, **dadurch gekennzeichnet, dass** das zweite Loch (68) einen Kragen (82) umfasst und der Haltering (78) ein elastisches Teil umfasst, wobei das elastische Teil bei einem Einführen der Kopfschraube (70) durch das zweite Loch (66) nach innen gebogen wird und nachdem das elastische Teil am Kragen (82) vorbei ist nach aussen gebogen wird, um eine Entfernung der Kopfschraube (70) zu verhindern.

12. Querverbinder nach Anspruch 11, **dadurch gekennzeichnet, dass** das elastische Teil ein Ende der Kopfschraube (70) umfasst mit einer Lippe und einer Anzahl Schlitzen.

13. Querverbinder nach Anspruch 1, **dadurch gekennzeichnet, dass** das Verbindungselement (22) am Steckelement einen Haken (90) umfasst und das Verbindungselement (26) am aufnehmenden Element einen Haken (90) umfasst.

14. Querverbinder nach Anspruch 13, **dadurch gekennzeichnet, dass** das laterale Ende des Steckelementes (16) und das laterale Ende des aufnehmenden Elementes (18) je in mit einem Gewinde versehenes Loch (92) und eine Klemmschraube (94) umfassen, welche zur Sicherung der Fixationselemente (12;14) an den entsprechenden Haken (90) des Steckelementes und des aufnehmenden Elementes (16;18) schraubbar im entsprechenden mit einem Gewinde versehenen Loch (92) aufnehmbar ist.

15. Querverbinder nach Anspruch 14, **dadurch gekennzeichnet, dass** jede Klemmschraube (94) ein erstes Ende (96) zur Aufnahme eines Werkzeuges zum Drehen der Klemmschraube (94), einen mit einem Gewinde versehenen zylindrischen ersten Körperabschnitt (100) und einen konischen zweiten Körperabschnitt (102) hat.

16. Querverbinder nach Anspruch 15, **dadurch gekennzeichnet, dass** der Haken (90) des Steckelementes (16) und der Haken (90) des aufnehmenden Elementes (18) je einen Endabschnitt (104) und einen gekrümmten Abschnitt (106) mit einem Krümmungsradius grösser als derjenige der Fixationselemente (12;14) umfassen.

17. Querverbinder nach Anspruch 16, **dadurch gekennzeichnet, dass** die Fixationslemente (12;14) zwischen dem konischen zweiten Körperabschnitt (102) der entsprechenden Klemmschraube (94) und einem Bereich nahe des Endabschnitts (104) des entsprechenden Hakens (90) eingeklemmt werden wenn der Querverbinder (10) an den Fixationselementen (12;14) fixiert wird.

18. Querverbinder nach Anspruch 1, **dadurch gekennzeichnet, dass** der Querverbinder (10) aus Titan, einer Titanlegierung oder rostfreiem Stahl hergestellt ist.

## Revendications

1. Transconnecteur (10) pour le couplage d'un premier et d'un deuxième éléments allongés de fixation spinale (12 ; 14) qui ont des orientations différentes, le transconnecteur (10) comprenant :
A) un élément mâle (16) comprenant un corps avec des extrémités latérales et médiales, un élément de liaison (22) associé à l'extrémité latérale et étant conçu et dimensionné de manière à recevoir l'un des éléments de fixation (12 ; 14) et une protubérance (24) au niveau de l'extrémité médiale, la protubérance (24) comprenant un corps avec un évidement (32) défini par des parois latérales et médiales (34 ; 36) ;
B) un élément femelle(18) comprenant un corps avec des extrémités latérales et médiales et présentant un trou taraudé (42), un élément de liaison (26) associé à l'extrémité latérale et étant conçu et dimensionné de manière à recevoir l'un des éléments de fixation (12 ; 14) et une cavité (28) avec une ouverture (30) au niveau de l'extrémité médiale qui est conçue et dimensionnée de manière à recevoir une partie de la protubérance de l'élément mâle (24) ;
**caractérisé en ce que** le transconnecteur comprend en outre
C) une vis de serrage (44) pour garantir la position et l'orientation de la partie de la protubérance de l'élément mâle dans la cavité (28) afin d'agencer des distances d'écart et des orientations différentes entre les premier et deuxième éléments de fixation (12 ; 14), la vis de serrage (44) reçue par filetage dans le trou taraudé (42) et ayant une première extrémité (48) pour recevoir un outil servant à tourner la vis de serrage (44) et une deuxième extrémité (46) qui peut venir en contact avec la protubérance (24) pour presser la protubérance (24) dans la cavité (28),
dans lequel l'interaction de la vis de serrage (44) avec la paroi médiale (36) de l'évidement (32) limite le déplacement de la partie de la protubérance de l'élément mâle dans la cavité (28) afin d'empêcher ainsi le découplage des éléments mâles et femelles (16 ; 18).

2. Transconnecteur selon la revendication 1 dans lequel l'évidement (32) a une largeur plus importante que la largeur de la deuxième extrémité (46) de la vis de serrage (44) afin de permettre la rotation de la protubérance (24) dans la cavité (28).

3. Transconnecteur selon la revendication 2 dans lequel la protubérance (24) a une forme essentiellement cylindrique avec un premier rayon et dans lequel l'évidement (32) a une surface supérieure courbée avec un deuxième rayon plus petit, dans lequel la deuxième extrémité (46) de la vis de serrage (44) s'étend dans l'évidement (32) afin de limiter la rotation de la protubérance (24) dans la cavité (28).

4. Transconnecteur selon la revendication 1 dans lequel la vis de serrage (44) comprend un corps cylindrique fileté (47) assurant la transition jusqu'à une pointe au niveau de la deuxième extrémité (46) de la vis de serrage (44).

5. Transconnecteur selon la revendication 1 dans lequel le corps de l'élément mâle comprend :
A) une extrémité de liaison (52) présentant une extrémité latérale avec l'élément de liaison de l'élément mâle (22) et une extrémité médiale ;
B) une liaison intermédiaire (54) présentant une extrémité médiale avec la protubérance (24) de l'élément mâle (16) et une extrémité latérale qui s'enclenche avec l'extrémité médiale de l'extrémité de liaison (52) ; et
C) un élément de verrouillage (56) pour fixer solidement l'extrémité de liaison (52) à la liaison intermédiaire (54).

6. Transconnecteur selon la revendication 5 dans lequel
A) l'extrémité médiale (60) de l'extrémité de liaison (52) comprend une première surface texturée (62) ;
B) l'extrémité latérale (58) de la liaison intermédiaire (54) comprend une deuxième surface texturée (64) conjuguée à la première surface texturée (62) ; et
C) la première surface texturée (62) peut tourner par rapport à la deuxième surface texturée (64) afin de s'adapter à la convergence ou la divergence entre le premier et deuxième élément de fixation (12 ; 14).

7. Transconnecteur selon la revendication 6 dans lequel les première et deuxième surfaces texturées (62 ; 64) comprennent un motif meulé en étoile.

8. Transconnecteur selon la revendication 6 dans lequel l'élément de verrouillage (56) comprend :
A) un premier trou (66) traversant l'extrémité médiale (60) de l'extrémité de liaison (52) ;
B) un deuxième trou (68) traversant l'extrémité latérale (58) de la liaison intermédiaire (54) connecté au premier trou (66) ; et
C) une vis à tête (70) insérable dans les premier et deuxième trous (66 ; 68).

9. Transconnecteur selon la revendication 8 dans lequel la vis à tête (70) présente une première extrémité (72) pour recevoir un outil afin de tourner la vis à tête (70) et une deuxième extrémité (76) avec une bague de retenue (78) pour empêcher l'extraction de la vis à tête (70) du deuxième trou (68).

10. Transconnecteur selon la revendication 9 dans lequel la vis à tête (70) présente un corps (84) avec un filetage (86) et le deuxième trou (66) est taraudé pour recevoir par filetage la vis à tête (70).

11. Transconnecteur selon la revendication 9 dans lequel le deuxième trou (66) comprend une collerette (82) et la bague de retenue (78) comprend un élément élastique, l'élément élastique fléchissant vers l'intérieur pendant l'insertion de la vis à tête (70) à travers le deuxième trou (66) et fléchissant vers l'extérieur une fois que l'élément élastique a passé la collerette (82) pour empêcher l'extraction de la vis à tête (70).

12. Transconnecteur selon la revendication 11 dans lequel l'élément élastique comprend une extrémité de la vis à tête (70) avec un rebord et une pluralité de fentes.

13. Transconnecteur selon la revendication 1 dans lequel l'élément de liaison de l'élément mâle (22) comprend un crochet (90) et l'élément de liaison de l'élément femelle (26) comprend un crochet (90).

14. Transconnecteur selon la revendication 13 dans lequel l'extrémité latérale de l'élément mâle (16) et l'extrémité latérale de l'élément femelle (18) comprennent chacune un trou taraudé (92) et une vis de serrage (94) reçue par filetage dans le trou taraudé respectif (92) pour serrer les éléments de fixation (12 ; 14) contre les crochets respectifs (90) des éléments mâles et femelles (16 ; 18).

15. Transconnecteur selon la revendication 14 dans lequel chaque vis de serrage (94) présente une première extrémité (96) pour recevoir un outil afin de tourner la vis de serrage (94), une partie d'un premier corps cylindrique taraudé (100), et une partie d'un deuxième corps conique (102).

16. Transconnecteur selon la revendication 15 dans lequel le crochet (90) de l'élément mâle (16) et le crochet (90) de l'élément femelle (18) comprennent chacun une partie de pointe (104) et une partie incurvée (106) qui a un rayon de courbure supérieur à celui des éléments de fixation (12 ; 14).

17. Transconnecteur selon la revendication 16 dans lequel les éléments de fixation (12 ; 14) sont serrés entre la partie du deuxième corps conique (102) de la vis de serrage respective (94) et un endroit près de la partie de pointe (104) du crochet respectif (90) lorsque le transconnecteur (10) est fixé sur les éléments de fixation (12 ; 14).

18. Transconnecteur selon la revendication 1 dans lequel le transconnecteur (10) est fait de titane, un alliage de titane ou d'acier inoxydable.
